(19) Europäisches Patentamt
European Patent Office
Office européen des brevets

(11) Publication number: 0 164 967 B1

(12) EUROPEAN PATENT SPECIFICATION

(45) Date of publication of patent specification: 12.09.90

(51) Int. Cl.$^5$: A 61 K 9/52, A 61 K 31/56

(21) Application number: 85303824.8

(22) Date of filing: 30.05.85

# (54) Pharmaceutical composition of sparingly soluble medicament and method for its preparation.

(30) Priority: 04.06.84 GB 8414221

(43) Date of publication of application: 18.12.85 Bulletin 85/51

(45) Publication of the grant of the patent: 12.09.90 Bulletin 90/37

(84) Designated Contracting States: AT BE CH DE FR IT LI LU NL SE

(56) References cited:
EP-A-0 061 217
DE-A-2 458 112
GB-A- 785 262
GB-A- 844 772

(73) Proprietor: STERWIN AG.
Zeughausgasse 9
CH-6300 Zug (CH)

(72) Inventor: Harrison, Paul Jonathan
7, Stott Street
Alnwick Northumberland (GB)
Inventor: Potter, Christopher John
2, Garden Terrace Whittingham
Alnwick Northumberland NE 66 4RD (GB)
Inventor: Langridge, John Richard
29 Arkle Court
Alnwick Northumberland (GB)

(74) Representative: Bankes, Stephen Charles Digby et al
BARON & WARREN 18 South End Kensington
London W8 5BU (GB)

EP 0 164 967 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a pharmaceutical composition in unit dosage form of a steroidal medicament having a solubility in water and aqueous media of less than 1 part by weight in 5000, preferably in 10,000, parts by weight at ambient temperatures.

The low solubility of a number of steroids having a polycyclic structure in aqueous media is a source of inconvenience and raises the overall cost of a course of treatment with any one of these drugs. These medicaments are synthetic materials having a steroidal unit in the molecule. One of the best known materials of this type is 17α-pregna-2,4-diene-20-yno [2,3-d]isoxazol-17-ol (compound A) which has been described as exceedingly insoluble. It has been given to patients in the form of a dry powder in a capsule. This material which inhibits the synthesis or release of pituitory gonadotrophins without exhibiting oestrogenic or progestational activity in patients has been administered in doses of 200 to 800 mg per day. The steroid may have been synthesised from naturally occurring ethisterone, supplies of which have been limited. It is therefore desirable that the steroid should be given to patients in a form in which it is most efficiently utilized by the body. Similar problems arise in the case of a number of other medicaments which comprise a steroidal unit in the molecule.

The low solubility of such materials in aqueous media, which media include the gastric juices, necessarily means that unless the concentration is pushed to the highest possible levels in the juices absorption into the plasma will be at a lower level than that which is theoretically attainable. This means a reduced bioavailability of the medicament and the enhanced risk of it passing along the intestinal tract without being further absorbed.

It is known that the solubility of some medicaments varies with the pH of the gastric juices but in the cases to which this invention relates change in pH with progress along the intestinal passage is unimportant as the solubility is low at all the relevant pH values. Thus the maintenance of a pH within a desired range is not a relevant consideration with the materials to which this invention relates (compare U.S. specifications Nos. 4361546, 4367217 and 4438091). As will be apparent as the present disclosure proceeds maximum solubility is secured by using the steroidal medicament in very finely divided form.

A form of sustained release formulation for aspirin and other medicaments having comparable solubility in water has been proposed in US—A—3388041. The solubility of aspirin in water is reported to be 1 gram in 300 ml of water at 25°C and the method is said to be applicable to other drugs having a solubility of less than 1%. The formulation consists of a two-layer tablet of which the first layer consists in a normal formulation of aspirin for rapid release whilst the second layer consists of aspirin particles which have been coated with a mixture of hydrophilic and hydrophobic ethers of cellulose. Thus the aim is an initial high threshold value of drug which is then substantially maintained for a period of time by the action of the gastric juices on the second layer of the tablet. The particles of medicament act as cores which are directly coated with the coating of cellulose ethers in this formulation.

The coating of pellets with coatings differing in thickness in admixture is proposed in US—A—2921883. These coatings are produced from a nontoxic solid hydroxylated lipid material and a nontoxic solid cellulose material which may be a cellulose ether or ester. Such material has been used to coat pellets comprising medicament in finely divided form which coatings are not sensitive to changes in pH and ready solubility of the medicament in the gastric juices is essential to the functioning of the formulation. The pellets used may have cores which are first coated with a solution of the medicament or medicaments and an adhesive which is conveniently gelatine and then with a coating powder and the procedure repeated until an adequate coating of the medicament has been applied to the nuclei after which the coating or coatings of lipid material and cellulosic material are applied. Thus the medicament is in the form of one or more thin coating layers.

GB—A—844772 discloses a granular pharmaceutical composition, to be made up into a suspension with water, made by coating coarse crystals of sugar with a water-soluble gum to render the surfaces of the crystals sticky and applying the active ingredient, such as tetracycline, to the sticky surfaces in finely divided form. This assists the eventual dispersion of the active ingredient, the sugar particles rapidly dissolving upon contact with water.

The present invention aims to provide a formulation of insoluble steroid medicaments in which the relative bioavailability of the medicaments containing a steroidal unit in the molecule is substantially enhanced.

Accordingly the invention provides a pharmaceutical composition comprising a plurality of beads together constituting a unit dose of a solid medicament and enclosed in containers formed of a gastric juice-soluble material, characterised in that said medicament is a steroidal compound having a solubility in water at ambient temperatures of less than 1 part in 5000 parts by weight, each of said beads comprising finely divided discrete solid steroidal particles, bound onto a particulate core by a binder soluble in water.

Each of the plurality of beads may comprise a core consisting of one or more nonpareils each of which have adherent thereto by the action of the binder a coating of finely divided discrete particles of said steroidal medicament. The thus prepared beads in unit dosage quantities are then used to fill capsules after which the capsules are closed and are then ready for administration.

A number of steroidal compounds having the indicated low solubility in water have the general formula

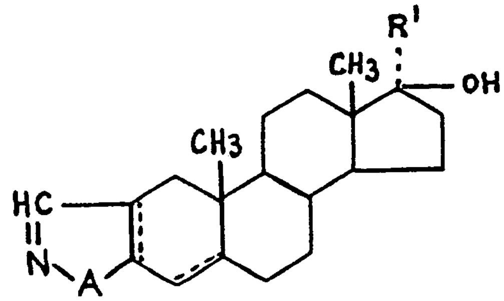

in which $R^1$ is an alkyl, alkenyl or alkynyl group having not more than 4 carbon atoms, A is an oxygen atom or a group of the formula

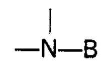

where B is a hydrogen atom, a para-fluorophenyl group or a double bond in the 3-position of the steroid skeleton. The production of such compounds is described in British Patent Specifications Nos. 905844, 911814 and 1,184,400. These compounds are steroidal oxazoles and pyrazoles.

A second group of steroidal compounds having the indicated low solubility in water have the general formula

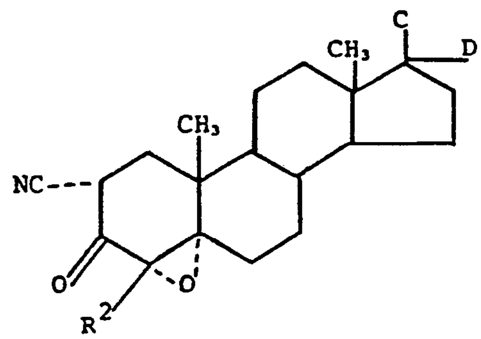

in which $R^2$ is hydrogen or methyl, C is hydrogen or hydroxyl and D is hydrogen or an alkyl, alkenyl or alkynyl group having not more than 4 carbon atoms. The production of such compounds is described in British Patent Specifications Nos. 1,123,730 and 2,010,278A. These compounds are steroidal carbonitriles.

Typical examples of compounds having the above general formulae are 17α-pregna-2,4-diene, 20-yno[2,3-d]-isoxazol-17-ol, 17β-hydroxy-17α-methylandrostano[3,2-c]-pyrazol, 17β-hydroxy-17a-ethynyl-4-androstano[3,2-c]-2′-(parafluorophenyl)pyrazol, 4α,5α-epoxy-17β-hydroxy-4,17-dimethyl-3-oxoandrostane-2α-carbonitrile and 4α 5α-epoxy-17β-hydroxy-3-oxoandrostane-2α-carbonitrile.

The binders used are those soluble in water. Preferred polymeric cellulosic binders for this purpose which are soluble in the gastric juices include hydroxypropylmethylcellulose. Other binders include the sugars derived from sugar syrups and the water-soluble polysaccharides. Binders which are only soluble in water and aqueous media at pH values above 7.0 merely act as fillers so far as the present invention is concerned. Non-cellulosic binders which may be used include polyvinylpyrrolidone and vinylpyrrolidone copolymers such as those with vinyl acetate. The core material may conveniently be an inorganic or organic nonpareil such as titania, talc, dibasic calcium phosphate, microcrystalline cellulose, lactose or a sugar/starch bead.

On account of the very low solubility of the steroidal medicaments used in the formulations of this invention in water and the consequent difficulty in wetting the particles thereof it is preferred in formulating the products to include a surface active agent. Examples of suitable surface active agents are sodium lauryl sulphate and polyoxyethylene ethers of mixed partial oleic esters of sorbitol anhydrides. Other non-ionic and anionic surface active agents having the necessary standard of purity may also be used.

The core is coated with discrete particles of the medicament in solid form, if necessary after grinding in order to obtain particles of sufficiently small size to be conveniently adhered to particles of core material. A convenient size for the core is that which will pass a 10 British standard mesh. The sizes of the core materials may be those which pass 10 to 60 British standard meshes. To adhere the particles of medicament to the core material a suspension in an aqueous solution of one or more of the binders is prepared. The aqueous solution used preferably contains a wetting agent to ensure that the particles are

thoroughly wetted. The resulting suspension is then used to coat the nonpareils in a coating column or coating pan and the coated particles are then dried using a current of warm air.

The beads are then weighed out in quantities constituting a single dose and each dose is then introduced into a gelatine capsule and the capsule then closed. Each capsule contains a unit dose consisting of beads in which discrete small particles of medicament adhere to a core. Adhesion of the particles is by means of the binder used and this may contain a small proportion of wetting agent.

The following examples illustrate the invention, all parts are by weight unless otherwise specified.

Example 1

The following are weighed out:

| | |
|---|---|
| Compound A | 100 parts |
| Hydroxypropyl methylcellulose | 50 parts |
| Nonpareils (sugar/starch) | 250 parts |

A solution is prepared of the hydroxypropylmethylcellulose in water and Compound A is dispersed therein. The nonpareils are placed in a coating column and the dispersion gradually added thereto whilst passing a current of warm air. After adding all the dispersion warm air is passed until the coated particles are dry. The coated particles are weighed out into portions of 400 mg and each portion fed into a No. 1 size gelatin capsule and capped.

Example 2

The following are weighed out:

| | |
|---|---|
| Compound A | 100 parts |
| Hydroxypropylmethylcellulose | 30 parts |
| Nonpareils (sugar/starch) | 100 parts |
| Sodium lauryl sulphate | 1 part |

A solution is prepared of hydroxypropylmethylcellulose and sodium lauryl sulphate and Compound A in small discrete particles is dispersed therein. The nonpareils are placed in a coating column and the dispersion gradually added thereto whilst passing a current of warm air. When all the dispersion has been introduced warm air is passed until the coated particles are dry. The dried coating particles are weighed out in portions each weighing 231 mg and each portion fed into a No. 2 size gelatin capsule and capped.

The new products, using capsules produced essentially in accordance with the procedure outlined in Example 2 but having the following composition (modified Example 2)

| | |
|---|---|
| Compound A | 100 parts |
| Hydroxypropylmethylcellulose 6 c.p.s. | 20 parts |
| Polyvinylpyrrolidone BP | 0.4 part |
| Sodium lauryl sulphate | 1.0 part |
| Non-pareils | 238.6 parts |

were tested to determine the bioavailability and compared with a known composition in capsule form containing compound A in dry powder form in admixture with conventional adjuvents as follows:

<u>Composition A</u>

| | |
|---|---|
| Compound A | 100 parts |
| Maize starch BP | 62 parts |
| Talc BP | 5 parts |
| Lactose BP | 62 parts |
| Magnesium stearate BP | 1 part |
| | 230 parts |

Thus capsules in accordance with Composition A contained 100 mg of Compound A in a total of 230 mg of filling.

A single capsule of one of the two kinds of composition outlined above was given to each of a number of volunteers selected at random and samples of plasma of each volunteer were taken prior to commencing the test and at various time intervals from 0.25 to 10 hours. Samples were taken after every 15 minutes during the first hour, then at 1.5, 2.0 and 2.5 hours after ingestion, then hourly from 3.0 to 6.0 hours and finally after 8 and 10 hours. A series of graphs were made from the data thus obtained, including graphs of the mean data. After a suitable interval the other composition was given respectively to each volunteer. Further graphs were prepared from the additional data thus obtained.

The following parameters were determined from the graphs: (a) maximum concentration of Compound A in the plasma (C max), (b) time to reach maximum concentration (T max), and (c) the area under the graph of plasma concentration against time up to the final observation 10 hours after ingestion (AUC). The following results were obtained.

| Composition | C max (ng/ml) | T max (h) | AUC (ng h/ml) |
|---|---|---|---|
| Composition A | 42.06 | 2.93 | 201.7 |
| Modified Example 2 | 65.35 | 2.35 | 269.0 |

The faster T max for the modified Example 2 indicates that for a more rapid release of medicament is obtained with the compositions of the present invention than with the hitherto known compositions: the decrease in time is approximately 20%. Moreover a higher concentration of medicament in human plasma is obtained when the same quantity of medicament is ingested.

Determination of AUC constitutes a measurement of the bioavailability of the administered medicament. When compositions containing equal weights of medicament are compared it is readily apparent that the bioavailability has increased. This constitutes an important economic advantage and could not be foreseen.

Thus the products of the invention give faster release of the insoluble steroiodal medicaments together with greater bioavailability thereof. Consequently less medicament is needed to secure a desired concentration of these steroidal medicaments in the plasma of patients.

**Claims**

1. A pharmaceutical composition comprising a plurality of beads together constituting a unit dose of a solid medicament and enclosed in containers formed of a gastric juice-soluble material, characterised in that said medicament is a steroidal compound having a solubility in water at ambient temperatures of less than 1 part in 5000 parts by weight, each said bead comprising finely divided discrete solid steroidal particles, bound onto a particulate core by a binder soluble in water.

2. A pharmaceutical composition according to claim 1, characterised in that the water solubility of said medicament is less than 1 part in 10,000 by weight.

3. A pharmaceutical composition according to claim 1 or claim 2, characterised in that said gastric juice-soluble material is in the form of a gelatin capsule.

4. A pharmaceutical composition according to any preceding claim, characterised in that the beads each comprise a core consisting of one or more nonpareils which have adherently bonded thereto, by the action of said binder, a coating of said solid steroidal particles.

5. A pharmaceutical composition according to any preceding claim characterised in that the beads further include a pharmacologically acceptable wetting agent.

6. A pharmaceutical preparation according to any preceding claim, characterised in that the medicament is selected from 17α-pregna-2,4-diene-20-yno[2,3-d]-isoxazol-17-ol, 17β-hydroxy-17α-methyl-androstano[3,2-c]-pyrazole, 17β-hydroxy-17a-ethynyl-4-androstano[3,2-c]-2′-(para-fluorophenyl) pyrazole, 4α, 5α-epoxy-17β-hydroxy-4,17-dimethyl-3-oxoandrostane-2α-carbonitrile and 4α 5α-epoxy-17β-hydroxy-3-oxoandrostane-2α-carbonitrile.

7. A pharmaceutical preparation according to any preceding claim, characterised in that the binder comprises hydroxypropyl methylcellulose, polyvinylpyrrolidone, a vinylpyrrolidone-vinyl acetate copolymer or a mixture thereof.

8. A pharmaceutical preparation according to any one of claims 1 to 6, characterised in that the binder is a water-soluble polysaccharide or a sugar derived from a sugar syrup.

9. A method of making a pharmaceutical preparation in unit dosage form of a solid steroidal medicament having a solubility in water at ambient temperatures of less than 1 part in 5000 parts by weight, characterised by forming beads by coating finely divided discrete particles of said steroidal compound, onto a particulate core material, with an aqueous diluent and a binder soluble in water, drying said beads and enclosing unit doses of said beads in containers formed of a gastric juice-soluble material.

10. A method according to claim 9, characterised by the steps of forming a suspension of the finely divided medicament particles in an aqueous solution of said binder, coating the suspension onto nonpareils constituting said particulate core material and drying the coated beads thus formed.

11. A method according to claim 10, characterised in that a pharmacologically acceptable wetting agent is included in said aqueous solution.

12. A method according to any one of claims 9 to 11, characterised in that the solid medicament is selected from 17α-pregna-2,4-diene-20-yno[2,3-d]-isoxazol-17-ol, 17β-hydroxy-17α-methylandrostano[3,2-c]-pyrazole, 17β-hydroxy-17a-ethynyl-4-androstano[3,2-c]-2'-(para-fluorophenyl) pyrazole, 4α, 5α-epoxy-17β-hydroxy-4,17-dimethyl-3-oxoandrostane-2α-carbonitrile and 4α 5α-epoxy-17β-hydroxy-3-oxo-androstane-2α-carbonitrile.

**Patentansprüche**

1. Pharmazeutische Zubereitung aus einer Mehrzahl von Perlen, die zusammen eine Dosiseinheit eines festen pharmazeutischen Wirkstoffs darstellen und in Behältern vorgesehen sind, die aus einem im Magensaft löslichen Material gebildet sind, dadurch gekennzeichnet, daß der pharmazeutische Wirkstoff eine Steroid-Verbindung ist, die in Wasser von Umgebungstemperatur eine Löslichkeit von weniger als 1 Gewichtsteil in 5000 Gewichtsteilen hat, wobei die Perlen aus feinverteilten einzelnen Teilchen der Steroid-Verbindung besteht, die auf einem teilchenförmigen Kern mittels eines in Wasser löslichen Bindemittels aufgebracht sind.

2. Pharmazeutische Zubereitung gemäß Anspruch 1, dadurch gekennzeichnet, daß die Wasserlöslichkeit des pharmazeutischen Wirkstoffs weniger als 1 Gewichtsteilchen in 10000 Gewichtsteilchen beträgt.

3. Pharmazeutische Zubereitung gemäß Anspruch 1 oder 2, dadurch gekennzeichnet, daß das magensaftlösliche Material in Form einer Gelatine-Kapsel vorliegt.

4. Pharmazeutische Zubereitung gemäß irgendeinem der vorstehenden Ansprüche, dadurch gekennzeichnet, daß jede Perle aus einem Kern aus einem oder mehreren nicht vergleichbaren Bestandteilen (non-pareils) besteht, die durch die Wirkung des Bindemittels eine Schicht aus diesen festen Steroid-Teilchen aufweisen.

5. Pharmazeutische Zubereitung gemäß irgendeinem der vorstehenden Ansprüche, dadurch gekennzeichnet, daß die Pugeln zusätzlich einen pharmakologisch annehmbaren oberflächenaktiven Stoff aufweisen.

6. Pharmazeutische Zubereitung gemäß irgendeinem der vorstehenden Ansprüche, dadurch gekennzeichnet, daß der pharmazeutische Wirkstoff ausgewählt ist aus der Gruppe 17-Pregna-2,4-dien-20-ino[2,3-d]-isoxazol-17-ol, 17-Hydroxy-17-methylandrostano[3,2-c]-pyrazol, 17-Hydroxy-17-ethinyl-4-androstano[3,2-c]-2'-(para-fluorphenyl)-pyrazol, 4,5-Epoxy-17-hydroxy-4,17-dimethyl-3-oxoandrostan-2-carbonitril and 4,5-Epoxy-17-hydroxy-3-oxoandrostan-2-carbonitril.

7. Pharmazeutische Zubereitung gemäß irgendeinem der vorstehenden Ansprüche, dadurch gekennzeichnet, daß das Bindemittel aus Hydroxypropylmethylcellulose, Polyvinylpyrrolidon, einem Vinylpyrrolidon-vinylacetat-Copolymerisat oder einem Gemisch aus mehreren solcher Produkte besteht.

8. Pharmazeutische Zubereitung gemäß irgendeinem der Ansprüche 1 bis 6, dadurch gekennzeichnet, daß das Bindemittel ein wasserlösliches Polysaccharid oder ein Zuckerderivat aus einem Zuckersyrup ist.

9. Verfahren zur Herstellung einer pharmazeutischen Zubereitung in Form einer Dosierungseinheit eines festen Steroid-Wirkstoffs mit einer Wasserlöslichkeit bei Umgebungstemperatur von weniger als 1 Gewichtsteil in 5000 Gewichtsteilen, gekennzeichnet durch Bildung von Perlen durch Aufschichtung von feinverteilten Einzelteilchen der Steroid-Verbindung auf ein teilchenförmiges Kernmaterial zusammen mit einem wässrigen Verdünnungsmittel und einem in Wasser löslichen Bindemittel, Trocknen dieser Perlen und Einbringung von Dosiseinheiten dieser Perlen in Behälter aus einem in Magensaft löslichem Material.

10. Verfahren gemäß Anspruch 9, gekennzeichnet durch die Stufen der Bildung einer Suspension von feinverteilten Wirkstoffteilchen in einer wässrigen Lösung des Bindemittels, Aufschichten der Suspension auf nicht vergleichbaren Bestandteilen (non-pareils), die dieses teilchenförmige Kernmaterial darstellen, und Trocknen der so gebildeten beschichteten Perlen.

11. Verfahren gemäß Anspruch 10, gekennzeichnet dadurch, daß die wässrige lösung einen pharmakologisch annehmbaren oberflächenaktiven Stoff enthält.

12. Verfahren gemäß irgendeinem der Ansprüche 9 bis 11, gekennzeichnet dadurch, daß der feste Wirkstoff ausgewählt ist aus der Gruppe 17-Pregna-2,4-dien-20-ino-2,3-d-isoxazol-17-ol, 17-Hydroxy-17-methylandrostan-(3,2-c)-pyrazol, 17-Hydroxy-17-ethinyl-4-androstano(3,2-c)-2'-(para-fluorphenyl)-pyrazol, 4,5-Epoxy-17-hydroxy-4,17-dimethyl-3-oxoandrostan-2-carbonitril und 4,5-Epoxy-17-hydroxy-3-oxo-androstan-2-carbonitril.

**Revendications**

1. Composition pharmaceutique comprenant de multiples perles constituant ensemble une dose unitaire d'un médicament solide et enfermées dans des enveloppes formées d'une matière soluble dans le suc gastrique, caractérisée en ce que le médicament est un composé stéroïdal possédant une solubilité dans l'eau, à la température ambiante, qui est inférieure à 1 partie dans 5.000 parties en poids, chacune des

perles étant constituée de particules stéroïdales distinctes de matière solide à l'état finement divisé, fixées sur un noyau particulaire à l'aide d'un liant soluble dans l'eau.

2. Composition pharmaceutique suivant la revendication 1, caractérisée en ce que la solubilité dans l'eau du médicament est inférieure à 1 partie dans 10.000 en poids.

3. Composition pharmaceutique suivant la revendication 1 ou 2, caractérisée en ce que la matière soluble dans le suc gastrique se présente sous la forme d'une capsule de gélatine.

4. Composition pharmaceutique suivant l'une quelconque des revendications précédentes, caractérisée en ce que les perles comportant chacune un noyau constitué d'un ou plusieurs nonpareils sur lesquels est fixé de manière adhérente, sous l'action du liant, un enrobage formé des particules stéroïdales de matière solide.

5. Composition pharmaceutique suivant l'une quelconque des revendications précédentes, caractérisée en ce que les perles comprenent en outre un agent mouillant acceptable sur le plan pharmacologique.

6. Composition pharmaceutique suivant l'une quelconque des revendications précédentes, caractérisée en ce que le médicament est choisi parmi le prégna-17α diène-2,4 yno-20 isoxazol[2,3-d] ol-17, l'hydroxy-17β(méthyl-17α androstano)pyrazole[3,2-c], l'hydroxy-17β éthynyl-17a androstano-4 (para-fluorophényl)-2' pyrazole[3,3-c], l'époxy-4α,5α hydroxy-17β diméthyl-4,17 (oxo-3 androstano) carbonitrile-2α et l'époxy-4α,5α hydroxy-17β (oxo-3 androstano) carbonitrile-2α.

7. Préparation pharmaceutique suivant l'une quelconque des revendications précédentes, caractérisée en ce que le liant est constitué par l'hydroxypropylméthylcellulose, la polyvinylpyrrolidone, un copolymère vinylpyrrolidone-acétate de vinyle ou un mélange de ceux-ci.

8. Préparation pharmaceutique suivant l'une quelconque des revendications 1 à 6, caractérisée en ce que le liant est un polysaccharide soluble dans l'eau ou un sucre obtenu à partir d'un sirop de sucre.

9. Procédé d'obtention d'une préparation pharmaceutique sous forme de dosage unitaire d'un médicament stéroïdal solide possédant une solubilité dans l'eau, à la température ambiante, qui est inférieure à 1 partie dans 5.000 parties en poids, caractérisé en ce qu'il consiste à former des perles en enrobant des particules distinctes, finement divisées, du composé stéroïdal, se trouvant sur une matière particulaire formant noyau, à l'aide d'un diluant aqueux et d'un liant soluble dans l'eau, à faire sécher ces perles et à enfermer des doses unitaires de celles-ci dans des enveloppes formées d'une matière soluble dans le suc gastrique.

10. Procédé suivant la revendication 9, caractérisé en ce qu'il comporte les opérations consistant à former une suspension des particules de médicament, finement divisées, dans une solution aqueuse du liant, à enrober de cette suspension les nonpareils constituant la matière particulaire du noyau et à faire sécher les perles enrobées ainsi formées.

11. Procédé suivant la revendication 10, caractérisé en ce qu'un agent mouillant acceptable sur le plan pharmacologique est inclus dans la solution aqueuse.

12. Procédé suivant l'une quelconque des revendications 9 à 11, caractérisé en ce que le médicament solide est choisi parmi le prégna-17α diène-2,4 yno-20 isoxazol[2,3-d] ol-17, l'hydroxy-17β(méthyl-17α androstano)pyrazole[3,2-c], l'hydroxy-17β éthynyl-17a androstano-4 (para-fluorophényl)-2' pyrazole[3,3-c], l'époxy-4α,5α hydroxy-17β diméthyl-4,17 (oxo-3 androstano) carbonitrile-2α et l'époxy-4α,5α hydroxy-17β (oxo-3 androstano) carbonitrile-2α.